# EUROPEAN PATENT SPECIFICATION

(11) **EP 2 886 125 B1**
(45) Date of publication and mention of the grant of the patent: **17.07.2019**
(21) Application number: 12882992.6
(22) Date of filing: 15.08.2012
(51) Int. Cl.: A61K 36/484, A61K 36/725, A61K 31/7076, A61P 25/24

(54) **PHARMACEUTICAL COMPOSITION INCREASING CYCLIC AMP CONTENT AND AVAILABILITY IN VIVO, AND PREPARATION METHOD THEREOF**
PHARMAZEUTISCHE ZUSAMMENSETZUNG ZUR IN-VIVO-ERHÖHUNG DES CAMP-GEHALTS UND DER CAMP-VERFÜGBARKEIT SOWIE HERSTELLUNGSVERFAHREN DAFÜR
COMPOSITION PHARMACEUTIQUE AUGMENTANT LA TENEUR ET LA DISPONIBILITÉ DE L'AMP CYCLIQUE IN VIVO, ET SON PROCÉDÉ DE PRÉPARATION

(43) Date of publication of application: 24.06.2015
(73) Proprietor: Chi, Yu-Fen, Yonghe City, Taipei County 234 (TW)
(72) Inventor: HSING, Chih-Kuang, New Taipei City Taiwan (TW)
(74) Representative: Bittner, Thomas L.
(86) International application number: PCT/CN2012/080191
(87) International publication number: WO 2014/026341

(56) References cited:
- EP-A1- 2 216 038
- EP-A1- 2 216 039
- EP-A1- 2 216 040
- CN-A- 101 450 070
- CN-A- 101 450 103
- CN-A- 101 450 120
- CN-A- 102 125 572
- US-A- 6 083 932
- YAO YANG ET AL: "Antidepressant Effects of Ginsenosides from Panax notoginseng", JOURNAL OF INTEGRATIVE AGRICULTURE, vol. 11, 1 March 2012 (2012-03-01), pages 483-488, XP055250502,
- WANG, WEIXIA ET AL.: 'Protective Effect and Mechanism of Ginsenosides on Central Nerve System of Animals' CHINESE JOURNAL OF INTEGRATED TRADITIONAL AND WESTERN MEDICINE vol. 25, no. 1, January 2005, pages 89 - 93, XP008176640
- SHI, QIUMING: 'Progresses in studies on pharmacologic effects of ginsenosides' CHINA PHARMACY vol. 21, no. 31, 2010, pages 2967 - 2969, XP008176661

## Description

### FIELD OF THE INVENTION

The present invention is related to a pharmaceutical composition, specifically to an oral medicine and health food.

### BACKGROUND OF THE INVENTION

Earl Wilbur Sutherland Jr., a scientist who was awarded a Nobel Prize in Physiology or Medicine in 1971, discovered a mechanism of cyclic adenosine monophosphate (cAMP) in cells. Edmond H. Fischer and Edwin G. Krebs, the scientists who were awarded a Nobel Prize in Physiology or Medicine in 1992, further discovered a mechanism of cAMP → PKA (protein kinase A, a cAMP dependent protein kinase) in cells. Eric Kandel, a scientist who was awarded a Nobel Prize in Physiology or Medicine in 2000, further discovered a mechanism of cAMP → PKA → CREB (cAMP response element-binding protein) in cells. It is apparent that there is an absolute and very significant relationship between the relative functions of cAMP and the physiology and medicine in human beings. For example, ever since Eric Kandel discovered that the mechanism for the formation of short-term memory and long-term memory is dependent on a "cAMP → PKA → CREB" signaling pathway (the second messenger signal transduction pathway) in cells and was awarded the Nobel Prize, scientists consider that these results are keys for inventing memory-enhancing drugs. Scientists have continuously endeavored and attempted to develop a drug to rapidly activate the cAMP → PKA → CREB signal transduction pathway in brain cells to enhance memory, improve learning ability, prevent and cure neurodegenerative diseases, such as amnesia, dementia, Alzheimer's disease, Parkinson's disease and so on, and further prevent and cure other diseases (such as depression) relevant to low cAMP content and low cAMP availability in organisms. However, there is no relevant medicine commercially available with suitable long-term use for humans, low side effects and high effectiveness, even though Earl Wilbur Sutherland Jr. was awarded the Nobel Prize in Physiology or Medicine over 30 years ago.

In the current technology, the marketed anti-depression drugs, e.g. selective serotonin reuptake inhibitors (SSRIs), serotonin-norepinephrine reuptake inhibitors (SNRIs), and norepinephrine-dopamine reuptake inhibitors (NDRIs), etc., inhibit the re-uptake of the first messenger neurotransmitters, such as 5-hydroxytryptamine (5-HT), norepinephrine (NE), dopamine (DA), etc., in depression patients, who have a lower concentration of the first messenger neurotransmitter than people without depression. After the concentration of the first messenger neurotransmitter and its combination with the receptor approached to normal levels, it may be possible to enable the production of a second messenger transmitter for cAMP in the patients' cells to be reached a normal level. However, high side effects and low remission rates make the anti-depression drugs undesirable. Therefore, there has never been a report announcing that healthy persons are taking depression drugs over a long time for the purpose of memory enhancement.

The National Institute of Mental Health (NIMH) of the National Institutes of Health (NIH) of the United States spent US$ 35 million and 6 years on remission rate research (STAR*D study) for 5 representative types of SSRI anti-depression drugs (Celexa®, Zoloft®, Wellbutrin®, Eflexor® and Buspar®) with over 2800 depression patients. The research report indicated that the remission rate for each drug is only about 30%, and it requires 6-7 weeks on average to relieve the symptoms of depression. Furthermore, the marketed anti-depression pharmaceuticals have different levels of side effects, such as increased suicide rate, headache, giddiness, vertigo, insomnia, hypersomnia, tinnitus, thirst, apocleisis, increased orexis, increased body weight, increased blood pressure, stomach upset, regurgitation nausea, emesis, dyspepsia, diarrhea, constipation, leg pain, skin rash, dither, convulsions, hyperhidrosis, edema, loss of libido and impotence, etc. In recent years, anti-depression pharmaceuticals, such as Prozac®, have become a serious social problem. In 2004, the Food and Drug Administration (FDA) of the United States further mandated that pharmaceutical companies had to revise product labels to clearly state the side effects and warnings on the instructions of 32 major anti-depression pharmaceuticals on the market, and emphasized to physicians and nurses that these pharmaceuticals might increase children's and adolescents' suicide rates.

Over the years, scientists in the worldwide pharmaceuticals industry have continuously endeavored to develop a safer and more effective pharmaceutical with low side effects, which can directly function on the receptor of the first messenger neurotransmitter to more rapidly increase the production and the availability of intracellular second messenger transmitters for cAMP, to prevent and cure the diseases related to a low intracellular cAMP level. Rolipram®, a phosphodiesterase 4 (PDE4) inhibitor, belongs to a type of post-receptor mechanism mediated drug, and the experimental results also indicate that Rolipram® has a significant anti-depression effect because the cAMP generated in the cells will be degraded by PDE4. That is to say, the availability of cAMP is increased by inhibiting PDE4. However, Roligram® is not broadly administered because Rolipram® may cause side effects such as severe emesis and so on.

The inventor developed a pharmaceutical composition that is prepared using three natural plants, i.e. ginseng, liquorice and jujuba, as the raw materials in order to solve the deficiencies in the prior art. The pharmaceutical composition not only increases the level of cAMP in cells, but also inhibits PDE4 to decrease the degradation of cAMP and increase the availability of cAMP, and increase the concentration of DA and NE neurotransmitters in the brain. The pharmaceutical composition is high safe for long-term use, is suitable for treating depression patients who need to take medicine for a long duration, and is suitable to prevent and cure diseases relevant to low intracellular cAMP levels and the deficiency of DA and NE neurotransmitters. However, the effective amount of the components that can increase the generation and availability of cAMP may be different in each batch of the natural plants because of differences in factors, such as the growth period, the place of origin, the harvest time and the preservation mode, climate change, temperature, rainwater, sun exposure, and so on. Therefore, the more definite of the active components, the more the amount of active components can be controlled and formulated, so that the efficacy and safety of the pharmaceutical composition maintain consistency batch by batch to improve the quality control (i.e. chemistry, manufacture and control (CMC)) of the pharmaceutical composition. Moreover, if the types of ginsenosides among the active components can be more definite, the range of the acquired raw materials can be broadened without reducing the supply. That is to say, in addition to the roots, stems and leaves of ginseng, those parts of other plants (such as *Panax notoginseng* and *Panax quinquefolius*) also contain various types of ginsenosides which can be used. Therefore, the inventor has continuously endeavored to search for more definitely-defined main effective components and their mechanisms on the basis of original research results in order to promote the quality control (i.e. CMC) of the pharmaceutical composition and broaden the origins of the acquired raw materials. He successfully developed a pharmaceutical composition having more definitely-defined main effective components, which contains ginsenosides Rg1 and Rb1, glycyrrhizic acid (or glycyrrhetinic acid) and jujuba cAMP, wherein the pharmaceutical composition is a multi-targeted post-receptor mechanism mediated drug, and the ginsenosides Rg1 and Rb1 are the main active components to enhance the expression of the brain-derived neurotrophic factor (BDNF). For example, European patent application no. 2 216 038 A1 of the inventor discloses a pharmaceutical composition to treat depression or an anxiety disorder, which includes the ginsenosides Rg1 and Rb1, glycyrrhizically-related acid, and/or a jujuba cAMP.

However, in addition to the ginsenosides Rg1 and Rb1, if the other types of ginsenosides contained in the roots, stems and leaves of the plants (such as ginseng, *Panax notoginseng, Panax quinquefolius* and so on) are used to solve the supply/insufficiency of the ginsenosides Rgl and Rb1 to achieve the effectiveness of the pharmaceutical composition above, the availability of the main active components in the natural plants can be further increased and the cost is reduced. In addition, the pharmaceutical composition above includes glycyrrhiza-related acids which may induce emesis symptoms if a person prone to emesis takes liquorice. This issue was confirmed by traditional Chinese physicians since ancient times.

It is therefore the inventor's invention to deal with the above limitations in the prior art.

### SUMMARY OF THE INVENTION

To overcome the deficiencies in the prior art, the present invention discloses a pharmaceutical composition including ginsenosides (Rg1, Rb1 and Re), glycyrrhizic acid and jujuba cAMP as the main active components to rapidly increase the content and the availability of cAMP in the body. In particular, the present invention discloses an oral pharmaceutical or a health food that can further increase the availability of the main active components in the natural plants, reduce the cost, enhance the cAMP/PKA/CREB signal transduction pathway by providing such products' stable quality, and enhance the expression of BDNF. In particular, the present invention provides said pharmaceutical composition for use in a method of treating neurodegenerative diseases and diseases caused by low cAMP expression in a patient, wherein said neurodegenerative diseases are selected from a group comprising amnesia, dementia, Alzheimer's disease and Parkinson's disease, and said diseases caused by low cAMP expression are selected from a group comprising heart failure, psoriasis and cancers, as it is claimed in the claims. In particular, the present invention provides said pharmaceutical composition wherein said pharmaceutical composition is manufactured as a drug, a health food or a nutrient for use in a method of treating neurodegenerative diseases and diseases caused by low cAMP expression in a patient as defined herein.

The scheme of the pharmaceutical composition in the present invention is the result of endeavors by the inventor. Because the prior art proved that the ginsenosides Rg1 and Rb1 can increase the BDNF expression in an organism, the ginsenosides Rg1 and Rb1 can be used to develop the main active components of the related pharmaceuticals. However, the ginsenosides Rg1 and Rb1 cannot be artificially synthesized using current technology, and hence they must be obtained from the roots, stems and leaves of natural plants, such as ginseng, *Panax notoginseng*, *Panax quinquefolius* and so on. Because there are more than 30 types of ginsenosides, to further increase the availability of the main active components in the natural plants and reduce the cost, the inventor incorporated ginsenoside Re with ginsenosides Rg1 and Rb1, and paired with glycyrrhizic acid (or glycyrrhetinic acid) and jujuba cAMP to form the main active components to prepare a pharmaceutical composition. After completing a substantial number of experiments, it was proven that the pharmaceutical composition of the present invention can increase the concentration of cAMP in the hippocampus, enhance the activity of PKA, promote the level of CREB phosphorylation, and inhibit the activity of PDE in the hippocampus in normal rats, 8 hours after they have been administered with the pharmaceutical composition. In a chronic repetitive stress experiment, the cAMP concentration, the PKA activity, the expression of CREB phosphorylation and the BDNF expression in the mice that were administered with the pharmaceutical composition were significantly higher than those of the mice in the control group which were not administered with the pharmaceutical composition, and the expressions of cAMP and PKA in the hippocampus, BDNF in the serum and the monoamine neurotransmitters (e.g. 5-HT, NE, DA and so on) in the hypothalamus of the "administered" mice were significantly higher than those in the "control" mice. Therefore, it was proven that the pharmaceutical composition in the present invention can rapidly increase the content and availability of cAMP in an organism. It is apparent that the pharmaceutical composition in the present invention demonstrates good effectiveness, increases the availability of the main active components in the natural plants and reduces the cost compared to the prior art, and the quality control (i.e. CMC) can be effectively performed. Furthermore, the pharmaceutical composition in the present invention is suitable for people worldwide because it is highly safe for long-term use, is suitable for treating depression patients who need long-term administration, and prevents and treats the diseases relevant to the low expressions of cAMP and BDNF and the insufficiency of the DA and NE neurotransmitters in the brain (e.g. neurodegenerative diseases such as amnesia, dementia, Alzheimer's disease, Parkinson's disease and so on, and diseases such as psoriasis, cancer and so on, where the patients have low cAMP expression in their bodies and cells). However, because the pharmaceutical composition includes a glycyrrhiza-related acid, it may induce emesis symptoms if a person prone to emesis takes liquorice (which is an issue confirmed by traditional Chinese physicians since ancient times). Nevertheless, traditional Chinese physicians also confirmed since ancient times that ginger is an excellent antiemetic food. Therefore, ginger powder or its extract is further added to the pharmaceutical composition of the present invention to improve the deficiencies in the prior art.

The present invention provides a pharmaceutical composition for use as claimed in the claims, wherein it is prepared using raw materials containing the active components, including ginsenosides (Rg1, Rb1 and Re), glycyrrhizic acid, jujuba cAMP. and others.

The pharmaceutical composition described in this specification and the claims of the present invention is the core content of the present invention. After the present invention is published, one skilled in the art can modify the drugs above using common techniques, and this is a regular technical activity for one skilled in the art. The invention is defined in the claims.

The above objectives and advantages of the present invention will become more readily apparent to those ordinarily skilled in the art after reviewing the following detailed descriptions and accompanying drawings.

### BRIEF DESCRIPTION OF THE DRAWINGS

Figure 1 is a flowchart showing a preparation method for a pharmaceutical composition in accordance with a first preferred embodiment of the present invention.
Figure 2 is a flowchart showing a preparation method for a pharmaceutical composition in accordance with a second preferred embodiment of the present invention.
Figure 3 is a flowchart showing a preparation method for a pharmaceutical composition in accordance with a third preferred embodiment of the present invention.
Figure 4 is a flowchart showing a preparation method for a pharmaceutical composition in accordance with a fourth preferred embodiment of the present invention.
Figure 5 is a diagram showing the level of cAMP in the hippocampus of rats, 8 hours after they were administered with the pharmaceutical composition.
Figure 6 is a gel electrophoresis photo showing the activity of cAMP-dependent PKA (lanes from left to right: Lanes 1-4: saline, Lanes 5-8: Paroxetine, Lanes 9-11: the embodiment 1, Lane 12: the positive control, and Lane 13: the negative control).
Figure 7 is a diagram showing the activity of cAMP-dependent PKA in the hippocampus of rats, 8 hours after they were administered with the pharmaceutical composition.
Figure 8 is a diagram showing the level of p-CREB in the hippocampus of rats, 8 hours after they were administered with the pharmaceutical composition.
Figure 9 shows the effect of embodiment 1 on the cAMP concentration in the hippocampus of repetitively stressed mice.
Figure 10 shows the effect of embodiment 1 on the PKA activity in the hippocampus of chronically stressed mice.
Figure 11 shows the effect of embodiment 1 on the CREP phosphoryplation in the hippocampus of chronically stressed mice.
Figure 12 shows the effect of embodiment 1 on the level of BDNF in the hippocampus of chronically stressed mice.

### DETAILED DESCRIPTION OF THE PREFERRED EMBODIMENTS

The present invention will now be described more specifically with reference to the following embodiments. It is to be noted that the following descriptions of preferred embodiments of this invention are presented herein for purpose of illustration and description only; they are not intended to be exhaustive or to be limited to the precise form disclosed.

In order to accomplish the purpose of the present invention, the technical schemes of the present invention are specifically described as follows.

The present invention discloses a pharmaceutical composition that rapidly increases the content and the availability of cAMP in an organism, wherein it is prepared using the main active components, including the ginsenosides (Rg1, Rb1 and Re), glycyrrhizic acid and jujuba cyclic adenosine monophosphate (jujuba cAMP).

### Example 1:

The pharmaceutical composition provided for use according to the present invention is prepared using the raw materials including ginsenosides (Rg1, Rb1 and Re), glycyrrhizic acid or glycyrrhetinic acid, and jujuba cAMP.

### Example 2:

The pharmaceutical composition provided for use according to the present invention is prepared using the raw materials including 2 - 26 parts by weight of ginsenosides (Rg1, Rb1 and Re), 3 - 48 parts by weight of glycyrrhizic acid or glycyrrhetinic acid, and 0.002 - 0.5 parts by weight of jujuba cAMP.

### Example 3:

The pharmaceutical composition provided for use of the present invention is prepared using the raw materials including 4 - 13 parts by weight of ginsenosides (Rg1, Rb1 and Re), 5 - 16 parts by weight of glycyrrhizic acid or glycyrrhetinic acid, and 0.01 - 0.1 parts by weight of jujuba cAMP.

### Example 4:

The pharmaceutical composition provided for use of the present invention further includes a ginger water extract.

### Example 5:

The pharmaceutical composition provided for use of the present invention can include pharmacologically acceptable carriers or additives, and can be manufactured in an oral pharmaceutical dosage form that is pharmaceutically known, such as a tablet, capsule, powder and so on.

### Example 6:

The pharmaceutical composition provided for use of the present invention can be manufactured as a pharmaceutical, health food or nutrient supplement for use in a method of treating neurodegenerative diseases and diseases caused by low cAMP expression in a patient as defined herein.

In order to accomplish the purposes of the present invention, the preparation methods for the pharmaceutical composition are described as follows.

### Method 1:

The extracts containing ginsenosides (Rg1, Rb1 and Re), glycyrrhizic acid (or glycyrrhetinic acid) and jujuba cAMP respectively extracted from ginseng, liquorice and jujuba act as the raw materials to be manufactured as the pharmaceutical composition to rapidly increases the content and availability of cAMP in an organism in the present invention. Alternatively, the prepared raw materials containing ginsenosides (Rg1, Rb1 and Re), glycyrrhizic acid (or glycyrrhetinic acid) and jujuba cAMP are manufactured as the pharmaceutical composition in the present invention.

### Method 2:

The pharmaceutical composition **provided for use of** the present invention is manufactured using the raw materials containing 2 - 26 parts by weight of ginsenosides (Rg1, Rb1 and Re), 3 - 48 parts by weight of glycyrrhizic acid (or glycyrrhetinic acid), and 0.002 - 0.5 parts by weight of jujuba cAMP.

### Method 3:

The pharmaceutical composition provided for use of the present invention is manufactured using the raw materials containing 4 - 13 parts by weight of ginsenosides (Rg1, Rb1 and Re), 5 - 16 parts by weight of glycyrrhizic acid (or glycyrrhetinic acid), and 0.01 - 0.1 parts by weight of jujuba cAMP.

### Method 4:

The pharmaceutical composition provided for use of the present invention further includes a ginger water extract.

### Method 5:

The pharmaceutical composition provided for use of the present invention can include pharmacologically acceptable carriers or additives, and can be manufactured in an oral pharmaceutical dosage form that is pharmaceutically known, such as a tablet, capsule, powder and so on.

### Method 6:

The health food or nutrient supplement for the use according to the present invention is manufactured using the raw materials in accordance with food management standards or methods of health food producing/manufacturing standards.

### THE PREFERRED EMBODIMENT

The present invention is further illustrated as follows by combining the figures with the preferred embodiments.

### Embodiment 1:

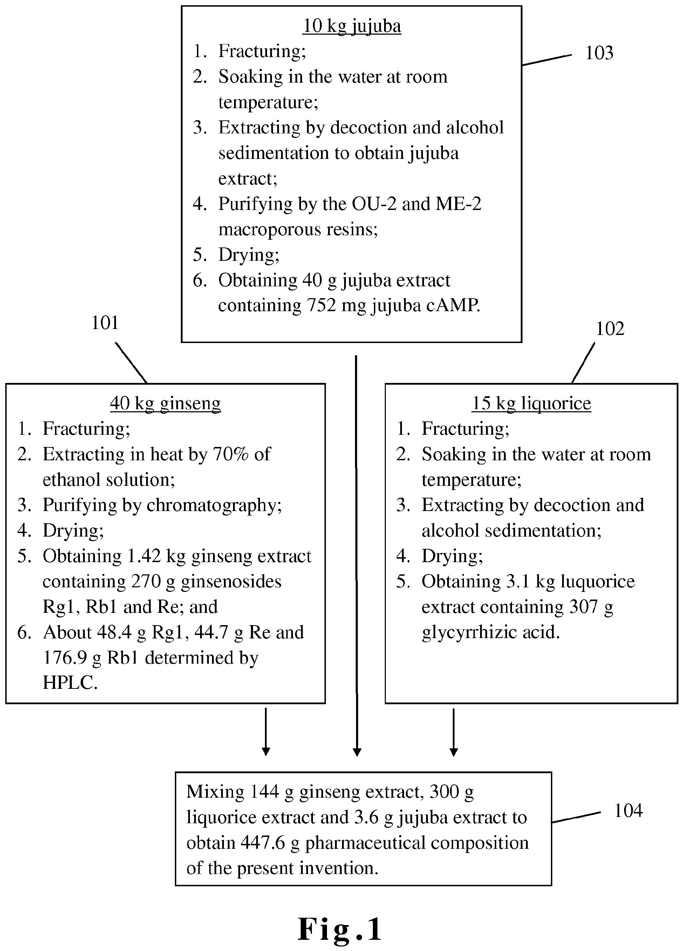

Please refer to Figure 1, which is a flowchart showing a preparation method for a pharmaceutical composition in accordance with a first preferred embodiment of the present invention. In Figure 1, 40 kg of ginseng is fractured followed by extraction in heat using a 70% ethanol solution. The ethanol extract is separated and purified using column chromatography, and dried to obtain 1.42 kg of the ginseng extract containing 270 g of ginsenoside Rg1+Rb1+Re (about 48.4 g of Rg1, about 176.9 g of Rb1 and about 44.7 g of Re) (step 101). Furthermore, 15 kg of liquorice is fractured followed by soaking at room temperature for 12 hours. The soaked liquorice is extracted using decoction and alcohol sedimentation, concentrated and dried to obtain 3.1 kg of the liquorice extract containing 307 g of glycyrrhizic acid (step 102). In addition, 10 kg of jujuba is fractured and then soaked in water at room temperature, and then the soaked jujuba is extracted using decoction and alcohol sedimentation to obtain the jujuba extract, which is further absorbed and separated using OU-2 and ME-2 macroporous resins sequentially, and dried. The jujuba extract (40 g) containing 0.752 g of jujuba cAMP is obtained and acts as the raw material for preparing the pharmaceutical composition of the present invention (step 103). Afterwards, 144 g of the ginseng extract, 300 g of the liquorice extract and 3.6 g of the jujuba cAMP obtained from the method above are pulverized and mixed to obtain 447.6 g of the pharmaceutical composition (containing 27.4 g of ginsenoside Rg1+Rb1+Re, 29.7 g of glycyrrhizic acid and 0.067 g of jujuba cAMP) in Example 1 in the present invention (step 104).

### Embodiment 2:

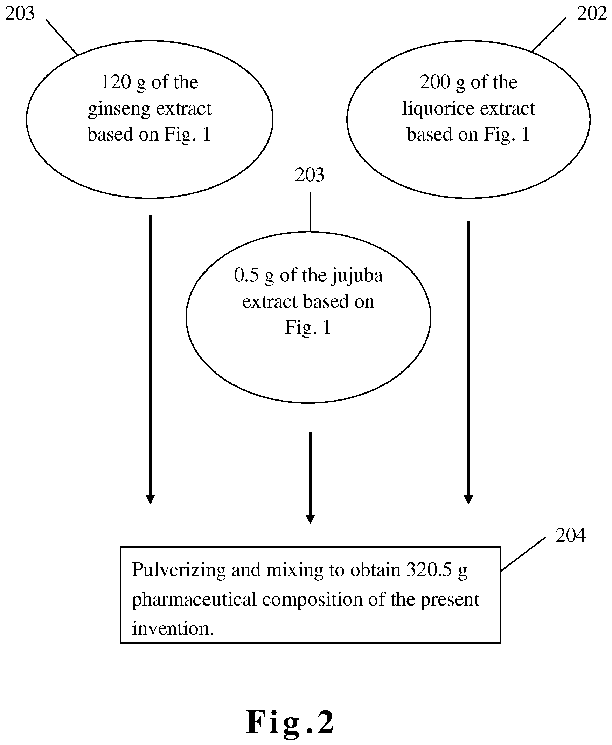

Please refer to Figure 2, which is a flowchart showing a preparation method for a pharmaceutical composition in accordance with a second preferred embodiment of the present invention. In Figure 2, 120 g of the ginseng extract (step 201), 200 g of the liquorice extract (step 202) and 0.5 g of the jujuba extract (step 203) obtained from Embodiment 1 are pulverized and mixed to obtain 320.5 g of the pharmaceutical composition (containing 22.8 g of ginsenoside Rg1+Rb1+Re, 19.8 g of glycyrrhizic acid and 0.009 g of jujuba cAMP) of Example 2 in the present invention (step 204).

### Embodiment 3:

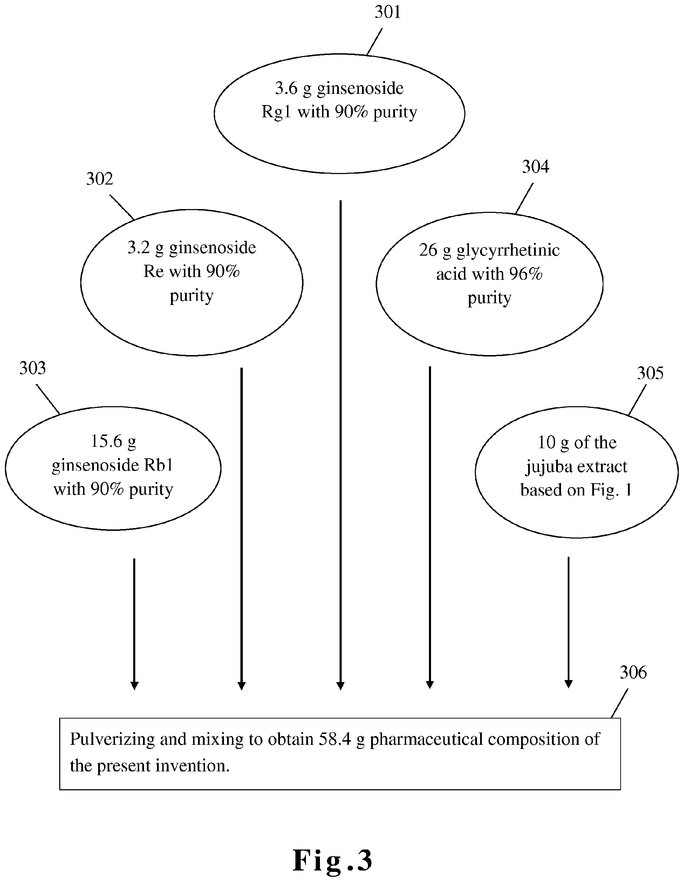

Please refer to Figure 3, which is a flowchart showing a preparation method of a pharmaceutical composition in accordance with a third preferred embodiment of the present invention. In Figure 3, 3.6 g of the prepared ginsenoside Rg1 with 90% purity (step 301), 3.2 g of the prepared ginsenoside Re with 90% purity (step 302), 15.6 g of the prepared ginsenoside Rb1 with 90% purity (step 303), 26 g of glycyrrhetinic acid with 96% purity (step 304) and 10 g of the jujuba extract obtained in Embodiment 1 (step 305) are pulverized and mixed to obtain 58.4 g of the pharmaceutical composition (containing 22.4 g of ginsenoside Rg1+Rb1+Re, 26 g of glycyrrhetinic acid and 0.188 g of jujuba cAMP) of Example 3 in the present invention (step 306).

### Embodiment 4:

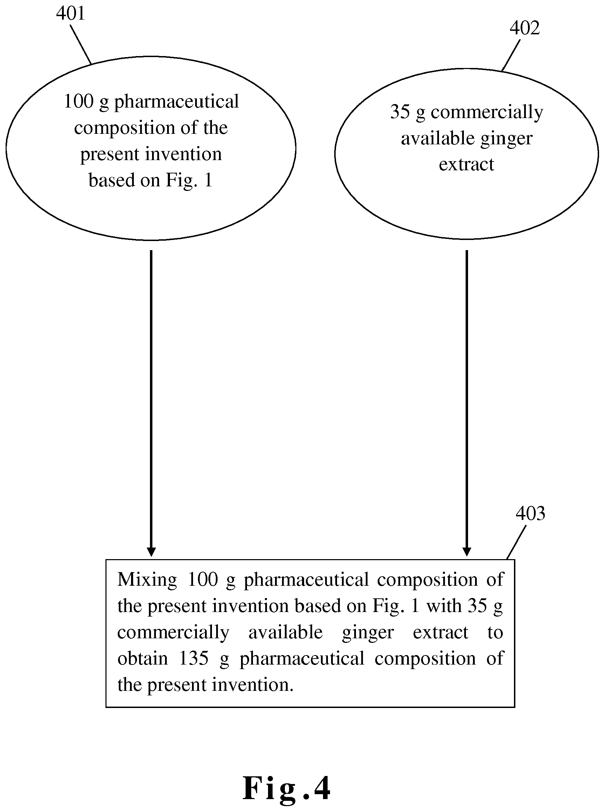

Please refer to Figure 4, which is a flowchart showing a preparation method for a pharmaceutical composition in accordance with a fourth preferred embodiment of the present invention. In Figure 4, 100 g of the pharmaceutical composition of Example 1 in the present invention obtained in Embodiment 1 (step 401) and 35g of commercially available ginger extract (step 402) are mixed to obtain 135 g of the pharmaceutical composition of Example 4 in the present invention (step 403).

**Experiment 1: An animal experiment for the influence on the cAMP/PKA/CREB signal transduction pathway 8 hours after normal rats were administered with the pharmaceutical composition of Embodiment 1.**

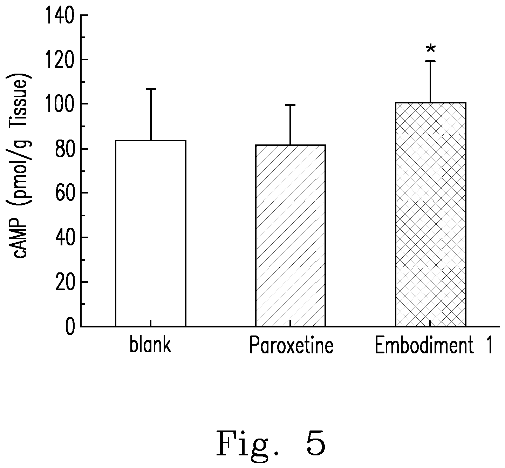

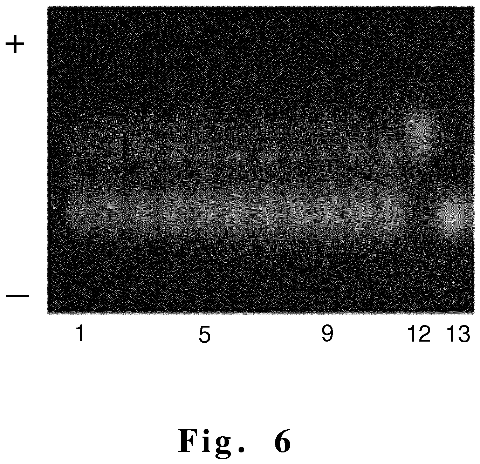

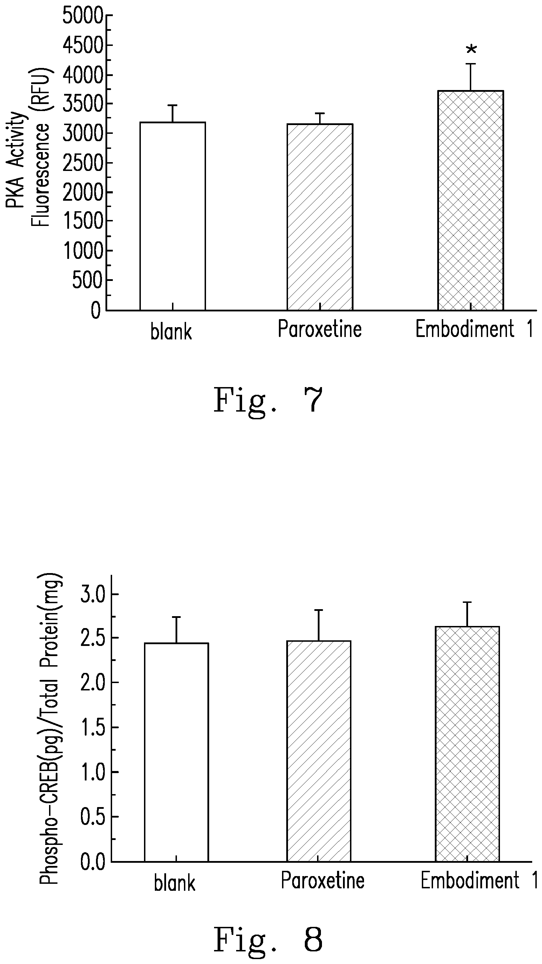

8 hours after normal rats were intragastrically administered with the pharmaceutical of Embodiment 1, their hippocampi were taken out. The variations in the levels of cAMP and phospho-CREB (p-CREB) in the hippocampus were determined using enzyme-linked immunosorbent assay (ELISA), the variations on the activities of the cAMP-dependent PKA were determined using bioluminescent assay, and the variations in the activities of PDE were determined using a fluorescence method. 8 hours after administering the pharmaceutical composition of Embodiment 1, it shows a molecular pharmacological mechanism wherein the cAMP concentration rises during a short time period, thereby enhance the cAMP-dependent PKA activity, promoting the level of the CREB phosphorylation and inhibiting the PDE activity in the hippocampus. The experimental data were statistically analyzed and plotted using Origin Pro 7.5 software.
1. Experimental materials:
1.1 Experimental animals: Seventy (70) healthy male SD rats with 180∼200 g of body weight were purchased from Vital River Laboratories (VRL), Beijing.
1.2 Reagents: positive control drug: anti-depression Paroxetine hydrochloride (lot number: 08030078, Zhong Mei Tianjin Smith Kline pharmaceuticals Co. Ltd.); Parameter™ Cyclic AMP Assay Kit, KGE002 (R&D Systems, Inc., U.S.); DuoSet IC Human/Mouse/Rat Phospho-CREB (S133) ELISA Kit, DYC2510-2 (R&D Systems, Inc., U.S.); PepTag® Assay for Non-Radioactive Detection of cAMP-Dependent Protein Kinase Kit, V5340 (Promega Corporation, U.S.); PDE-Glo™ Phosphodiesterase Assay Kit, V1361 (Promega Corporation, U.S.); Pierce™ BCA Protein Assay Kit, 23227, (Pierce Biotechnology, U.S.); the standards such as cAMP and adenosine were purchased from the National Institutes for Food and Drug Control, China; the chemicals such as NaH₂PO₄, Na₂HPO₄, KH₂PO₄, KCl, NaCl, MgCl₂, Tris Base, Tris-HCl and so on were the biochemical reagents for the cell culture grade and purchased from Sigma, U.S.; the reagents such as E-64, aprotinin, leupeptin, pepstatin A, phenylmethylsulfonyl fluoride (PMSF), NaF, ethylenediaminetetraacetic acid (EDTA), ethylene glycol tetraacetic acid (EGTA), dithiothreitol (DTT), NaVO₄, sodium pyrophosphate, agarose, glycerol and so on were a high purity grade were purchased from BioBasic Inc., Canada; acetonitrile and methanol (the HPLC grade, Merck & Co., Inc., Germany); ultrapure water (Milli-Q® pure water); and the pharmaceutical composition of Embodiment 1.
1.3 Experimental equipment: FlexStation® 3 multi-mode microplate reader (Molecular Devices Corp., U.S.); Waters® 600E high-performance liquid chromatography (including the quaternary pump, on-line degasser, autosampler, column oven, and UV detector; Waters Corp., U.S.); refrigerated centrifuger (Backmen Coulter, Inc., U.S.); electronic ultrasonic homogenizer (Ultrasound Technology Inc., U.S.); electrophoresis apparatus (Beijing Liuyi Instrument Factory, China); gel imager (SYN GENE); ultra-low temperature freezer (SANYO Electric Co., Ltd., Japan); and mLine single-channel pipette and 8-channel pipette (Biohit, Finland).
2. Administration: After adaptive feeding for three days, the rats were randomly divided into three groups, and labeled as follows: group A - saline, group B - Paroxetine, and group C - Embodiment 1. Paroxetine hydrochloride tablets were crushed and formulated into a suspension at a specific concentration using ultrapure water, and the intragastrically administered dose was 5 mg/kg per rat. The pharmaceutical composition of Embodiment 1 was formulated to a solution at a specific concentration using ultrapure water, and the intragastrically administered dose was 50 mg/kg per rat. The rats in the saline group were administrated with the equivoluminal 0.9% saline. All the rats were weighed and marked with picric acid prior to administration, and all drugs were administered after heating at 37°C for 30 minutes in advance.
3. Collection of tissues: 8 hours after the experimental animals in groups A, B and C were administered, the rats were anesthetized with ethyl ether and sacrificed by bleeding from their femoral arteries. The brains were collected on ice via the removal of the head, and the hippocampus was harvested and divided into three parts. Each part was placed in a 1.5-mL cryotube with a colored screw cap and labeled in advance, accurately weighed, rapidly frozen in liquid nitrogen for 15 minutes, and then stored in the refrigerator at -80°C for further use.
4. Sample determination:
4.1 Determination of the level of cAMP in the rat's hippocampus: The sample of hippocampus tissue was unfrozen and washed with a small amount of saline, and then the cell lysis solution (where the concentrated solution was diluted 5 times for use) provided by the kit was added at a ratio of 1:20 (weight: volume (g: mL)). The sample was homogenized in an electronic ultrasonic homogenizer for 30 seconds, centrifuged at 10000 rpm for 5 minutes at 4°C. The supernatant was placed in a 1.5-mL colored Eppendorf and labeled in advance and stored in an ice box for testing. The sample was determined using the Parameter™ Cyclic AMP Assay ELISA kit (R&D Systems, Inc., U.S.). The sample was defrosted to room temperature, and the level of cAMP in the sample was determined using competitive ELISA. The value of optical density (OD) of the sample was determined at 450 nm using the multi-mode microplate reader, and the level of cAMP in the sample was calculated according to the standard curve.
4.2 Determination of the activity of cAMP-dependent PKA in the rat's hippocampus: The sample of hippocampus tissue was unfrozen and washed with a small amount of saline, and then the PKA extraction buffer (which was prepared according to the formulation in the manufacturer's manual) was added at a ratio of 1:10 (weight: volume (g: mL)) ratio. The sample was homogenized in an electronic ultrasonic homogenizer for 30 seconds, centrifuged at 10000 rpm for 5 minutes at 4°C. The supernatant was transferred to a 1.5-mL colored Eppendorf and labeled in advance and stored in an ice box for testing. The sample was determined using the PepTag® Assay for Non-Radioactive Detection of cAMP-Dependent Protein Kinase Kit (Promega Corporation, U.S.). According to the instruction manual in the kit, the pre-mixed reagent was added to 200-µL 8-tube PCR tubes and labeled in advance, and 9 µL of each sample was added to the corresponding tube. The tube was vortexed, centrifuged and reacted at room temperature for 30 minutes, and then placed in a PCR machine to inactivate the enzyme at 98°C for 5 minutes. A positive control and a negative control were determined together with the tested samples according to the instruction manual. A 0.8% agarose gel was prepared, and 10 µL of each sample after the enzymatic denaturation was injected into the hole of the agarose gel, and electrophoresis was run at 100 V and 130 mA for 30 minutes. The electrophoresis buffer was 50 mM Tris-HCl (pH 8.0) buffer. After electrophoresis, the agarose gel was removed from the apparatus and photographed under the gel imager. The agarose gel was placed on the UV analyzer, the phosphorylated PepTag A1 Peptide spots were cut and placed in a 1.5-mL cryotube with a colored screw cap and labeled in advance. The agarose gel was heated to melt, and pure water was added to a volume of 250 µL. The hot agarose of 125 µL was quickly transferred to another 1.5-mL Eppendorf and labeled in advance, and 75 µL of the gel solubilization solution and 50 µL of glacial acetic acid were added. The mixture was vortexed, and 200 µL of the mixture was added to a 96-well microtiter plate. The agarose toward the positive electrode in the negative control was the blank. Fluorescent analysis was conducted on a multi-mode microplate reader, and the excitation wavelength of 586 nm and the emission wavelength of 592 nm were set. The fluorescent intensity of the sample refers to the PKA activity.
4.3 Determination of the level of p-CREB in the rat's hippocampus: The samples were determined using DuoSet IC Human/Mouse/Rat Phospho-CREB (S133) ELISA Kit (R&D Systems, Inc., U.S.). The sample of hippocampus tissue was unfrozen and washed with a small amount of saline, and then the tissue homogenization solution (which was prepared according to the IC Diluent #6 in the manufacturer's manual) was added at a ratio of 1:20 (weight: volume (g: mL)). The mixture was homogenized in an electronic ultrasonic homogenizer for 30 seconds, centrifuged at 10000 rpm for 5 minutes at 4°C, the supernatant was transferred to a 1.5-mL colored Eppendorf and labeled in advance and stored in an ice box for testing. The level of p-CREB in the sample was determined using the sandwich ELISA according to the instruction manual in the kit. The OD value of the sample was determined at 450nm using the multi-mode microplate reader, and the level of p-CREB in the sample was calculated according to the standard curve.
4.4 Determination of the total protein in the rat's hippocampus: In order to more accurately calculate the amount of p-CREB protein per milligram of protein contained in the sample, it is essential to determine the amount of the total protein in the sample. The supernatant after the tissue was homogenized and centrifuged in the p-CREB determination experiment was diluted with PBS 25 times to form the test samples. In accordance with the instruction manual in the Pierce™ BCA Protein Assay Kit, the OD value of the sample was determined at 450nm using the multi-mode microplate reader, and bovine serum albumin (BSA) is the standard. The amount of total protein in the sample was determined according to the standard curve.
4.5 Determination of the PDE activity: The influence of the pharmaceutical composition of Embodiment 1 on the PDE activity in the rat's hippocampus was determined by bioluminescent assay using a PDE-Glo™ Phosphodiesterase Assay Kit (Promega Corporation, U.S.).
4.5.1 Preparation of the medical solution: The pharmaceutical composition of Embodiment 1 was prepared to the concentrations of 0.02 mg/ml, 0.05 mg/ml and 1.0 mg/ml.
4.5.2 Preparation of the sample: After a specific amount of the hippocampus tissue was washed with a small amount of saline, and the PDE-Glo™ Reaction Buffer (Tris-HCl of 40 mM, MgCl₂ of 10 mM, and bovine serum albumin (BSA) of 0.1 mg/mlsupplemented with PMSF of 1 mM, leupetin of 2 µM/mL, aprotinin of 2 µM/mL, and E-64 of 2 µM/mL) was added at a ratio of 1:10 (weight: volume (g: mL)). The mixture was homogenized using an electronic ultrasonic homogenizer and centrifuged at 10000 rpm for 5 minutes at 4°C, and the supernatant was the enzymatic solution for use.
4.5.3 Protocol of the bioluminescent assay: The protocol was conducted according to the method provided in the instruction manual. As to the enzymatic reaction solution, 1 µL medical solution was added to 1.5 µL enzymatic solution to a total volume of 2.5 µL. The substrate solution of 2.5 µL containing 2 µmol cAMP was added to 2.5 µL of the mixture, and the reaction mixture was mixed and reacted at 37°C for 30 minutes. Next, 2.5 µL of the stop solution containing 3-isobutyl-1-methylxanthine (IBMX) (which is a strong PDE inhibitor) was added and mixed. A test solution of 2.5 µL was added and mixed, and the reaction mixture was reacted at room temperature for 20 minutes. Finally, 10 µL of the luminescent reagent was added to the reaction mixture, which then was reacted at room temperature for 10 minutes. The bioluminescence was determined using the multi-mode microplate reader.
5. Experimental results:
5.1 The level of cAMP in the rat's hippocampus: The cAMP concentration in the homogenate of the rat's hippocampus was determined using the ELISA assay, and the level of cAMP contained in the hippocampus tissue was obtained by dividing the weight of the measured tissue sample by the concentration of cAMP, and is presented as pmol / g tissue (referring to Figure 5).
   Please refer to Figure 5. The level of cAMP in the rat's hippocampus tissue in the Embodiment 1 group significantly increased compared to the saline group and the Paroxetine group (*P <0.05, n = 10).
5.2 The activity of cAMP-dependent PKA in the rat's hippocampus: After the enzymatic reaction, the samples were resolved using agarose gel electrophoresis, developed using the gel imager, and roughly analyzed.
   Please refer to Figure 6, the phosphorylated A1 peptide has a negative charge and migrates toward the positive electrode, while the unphosphorylated A1 peptide has a positive charge and migrates toward the negative electrode. These two peptides were separated from each other. When the brightness of the phosphorylated A1 peptide spot (which migrates toward the positive electrode) is stronger, this indicates that the level of phosphorylation and the activity of cAMP-dependent PKA are higher in the sample. It is shown in Figure 6 that the brightness of the spot in the Embodiment 1 group is stronger than those in the saline group and the Paroxetine group.
   The spots on the agarose gel were cut and melted, and the volume of the melted gel was determined. The activity of cAMP-dependent PKA in the rat's hippocampus was determined using the fluorescent assay and was presented as fluorescent intensity (referring to Figure 7).
   Please refer to Figure 7. 8 hours after administration, the activity of cAMP-dependent PKA in the rat's hippocampus in the Embodiment 1 group is significantly increased compared to the blank control group and the Paroxetine group (*P <0.05, n = 10).
5.3 The level of p-CREB in the rat's hippocampus: The concentration of total protein in the homogenate of the rat's hippocampus sample was determined using the BCA method to assess the amount of p-CREB per µg of total protein. Next, the concentration of p-CREB in the homogenate of the rat's hippocampus sample was determined using the sandwich ELISA assay, and p-CREB (pg) / total protein (µg) referred to the level of p-CREB in the rat's hippocampus (referring to the data in Figure 8).
   Please refer to Figure 8. 8 hours after administration, the level of p-CREB in the rat's hippocampus in the Embodiment 1 group significantly increased compared to the saline group and the Paroxetine group (n = 10).
5.4 The influence of the pharmaceutical composition on the PDE activity in the rat's hippocampus: The PDE activity in the rat's hippocampus and the inhibition against the PDE activity after *in vitro* administration were determined using the bioluminescent assay. The luminous intensity refers to the PDE activity. Compared with the control group, the higher luminous intensity indicates higher PDE activity, and the lower luminous intensity indicates that the PDE activity was inhibited. The measured results show that the pharmaceutical composition (0.5mg/ml) in the Embodiment 1 group significantly inhibited the PDE activity in the rat's hippocampus compared to the blank control group (*P <0.05, n = 4).
6. Conclusion:
   (1) 8 hours after the intragastric administration to the rats, compared with the saline group and the Paroxetine group, the level of cAMP in the rat's hippocampus in the Embodiment 1 group significantly increased, the cAMP-Dependent PKA activity therein was significantly enhanced, and the level of p-CREB therein also increased.
   (2) The experiments demonstrate that the pharmaceutical composition of Embodiment 1 can perform its pharmacological functions via the second messenger cAMP signaling pathway, and can rapidly initiate the cAMP-PKA-CREB (p-CREB) pathway 8 hours after administration (where the significant difference (*P <0.05) was done by comparison with the saline group and the Paroxetine group).
   (3) 8 hours after administration, the positive control drug, Paroxetine, cannot initiate the cAMP-PKA-CREB (p-CREB) pathway.
   (4) The experimental result also indicates that the PDE activity can be significantly inhibited by a medium dose of the pharmaceutical composition in Embodiment 1. Because PDE is an inactivated enzyme for cAMP, the inhibition of PDE can cause the increased level of cAMP in the rat's hippocampus.

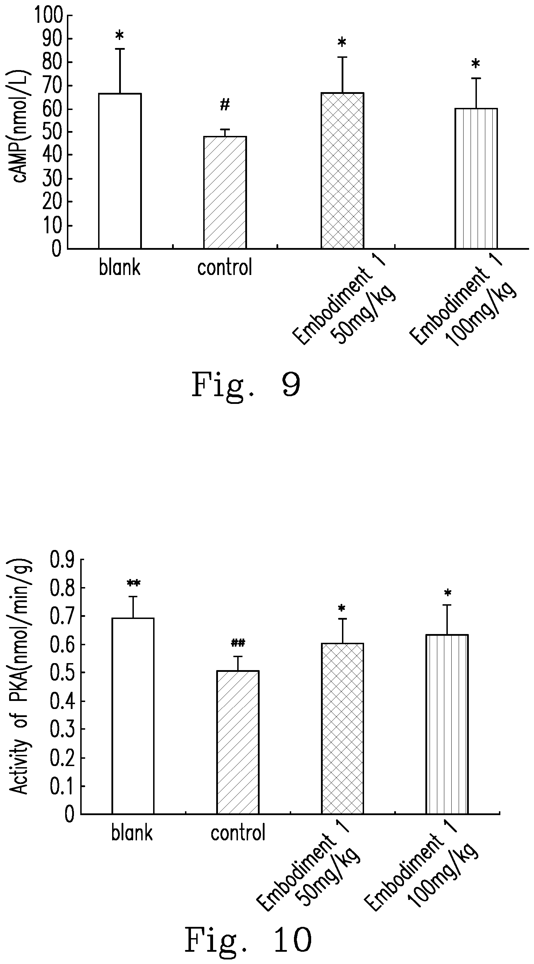

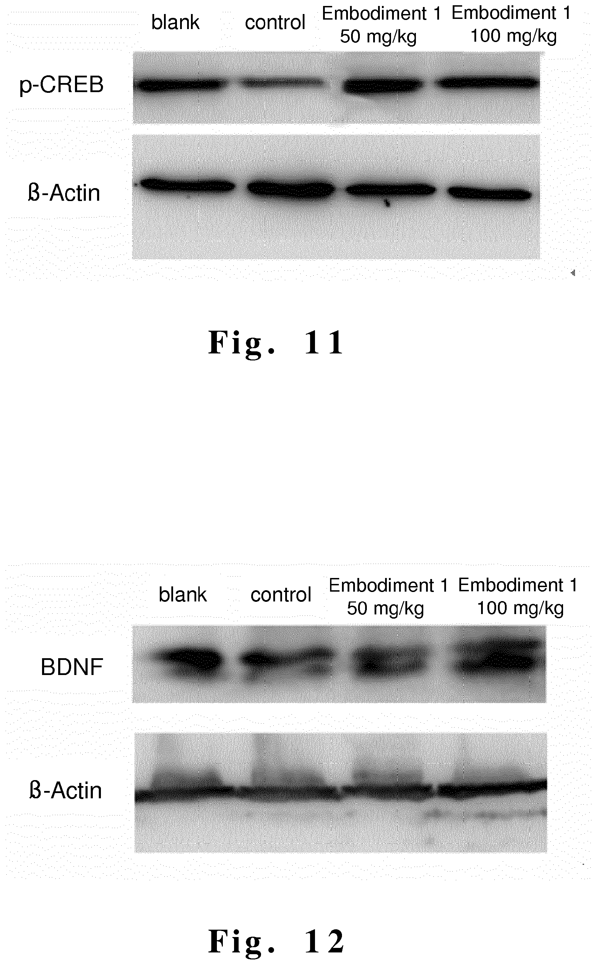

**Experiment 2: An animal experiment for the influence to the cAMP/PKA/CREB signal transduction pathway and the BDNF expression after the chronically stressed mice were administered with the pharmaceutical composition of Embodiment 1 for 10 days.**
1. The effect of the pharmaceutical composition of Embodiment 1 on the cAMP concentration of the hippocampus in the repetitively stressed mice (referring to Figure 9):
   The results in Figure 9 show that the cAMP concentration in the hippocampus of the mice that were administered with the pharmaceutical composition of Embodiment 1 is significantly higher than that in the control group without administration (*P <0.05).
2. The effect of the pharmaceutical composition of Embodiment 1 on the PKA activity of the hippocampus in the repetitively stressed mice (referring to Figure 10):
   The results in Figure 10 show that the PKA activity in the hippocampus of the mice that were administered with the pharmaceutical composition of Embodiment 1 is significantly higher than that in the control group without administration (*P <0.05).
3. The effect of the pharmaceutical composition of Embodiment 1 on the CREB phosphorylation of the hippocampus in the repetitively stressed mice (referring to Figure 11):
   The results in Figure 11 show that the expression of the CREB phosphorylation in the hippocampus of the mice that were administered with the pharmaceutical composition of Embodiment 1 is significantly higher than that in the control group without administration.
4. The effect of the pharmaceutical composition of Embodiment 1 on the BDNF expression of the hippocampus in the repetitively stressed mice (referring to Figure 12):

The results in Figure 12 show that the BDNF expression in the hippocampus of the mice that were administered with the pharmaceutical composition of Embodiment 1 is significantly higher than that in the control group without administration.

**Experiment 3: An animal experiment for the influence on the expressions of hippocampal cAMP and PKA, serum BDNF, and hypothalamic NE, DA and 5HT after the chronically stressed rats were administered with the pharmaceutical composition of Embodiment 1 for 21 days.**
1. The effect of the pharmaceutical composition of Embodiment 1 on the cAMP concentration of the hippocampus and cortex in the chronically stressed rats (referring to Table 1).

**Table 1. The cAMP expression in the hippocampus and cortex of the rats in each group.**

| Group | Sample size | Dose (mg/kg/d) | Hippocampus (pmol/ml) | Cortex (pmol/ml) |
|---|---|---|---|---|
| Normal | 8 | - | 72.650±2.9017 | 67.650±4.0715 |
| Control | 8 | - | 64.950±7.6459 | 57.825±5.8004 |
| High dose of Embodiment 1 | 8 | 60 | 69.887±7.2155 | 66.850±5.0844 |
| Medium dose of Embodiment 1 | 8 | 30 | 71.400±6.0233 | 66.975±6.6626 |
| Low dose of Embodiment 1 | 8 | 15 | 69.113±5.4286 | 65.388±8.0920 |

2. The effect of the pharmaceutical composition of Embodiment 1 on the PKA level of the hippocampus in the chronically stressed rats (referring to Table 2).

**Table 2. The PKA level in the hippocampus of the rats in each group.**

| Group | Sample size | Dose (mg/kg/d) | Hippocampus (pmol/ml) |
|---|---|---|---|
| Normal | 8 | - | 26.4988±2.75623 |
| Control | 8 | - | 21.4936±3.01990 |
| High dose of Embodiment 1 | 8 | 60 | 25.8763±3.48088 |
| Medium dose of Embodiment 1 | 8 | 30 | 25.9150±4.77464 |
| Low dose of Embodiment 1 | 8 | 15 | 26.0025±4.87687 |

3. The effect of the pharmaceutical composition of Embodiment 1 on the serum BDNF level in the chronically stressed rats (referring to Table 3).

**Table 3. The serum BDNF level of the rats in each group**

| Group | Sample size | Dose (mg/kg/d) | BDNF (pg/ml) |
|---|---|---|---|
| Normal | 8 | - | 111.00±45.28 |
| Control | 8 | - | 72.57±19.49 |
| Low dose of Embodiment 1 | 8 | 15 | 102.90±18.70 |
| Medium dose of Embodiment 1 | 8 | 30 | 110.14±31.64 |
| High dose of Embodiment 1 | 8 | 60 | 91.53±39.93 |

4. The effect of the pharmaceutical composition of Embodiment 1 on the expression of monoamine neurotransmitters of the hypothalamus in the chronically stressed rats (referring to Table 4).

**Table 4. The expression of monoamine neurotransmitters in the hypothalamus of the rats in each group**

| Group | Sample size | Dose mg/kg/d | 5HT (ng/g wet tissue) | NE (ng/g wet tissue) | DA (ng/g wet tissue) |
|---|---|---|---|---|---|
| Normal | 11 | - | 1038.03±458.63 | 4254.74±1334.95 | 546.04±329.18 |
| Control | 11 | - | 324.22±89.44 | 2572.61±707.43 | 229.40±109.74 |
| Low dose of Embodiment 1 | 11 | 15 | 990.45±261.38 | 4128.42±1608.56 | 477.27±274.74 |
| Medium dose of Embodiment 1 | 11 | 30 | 857.52±273.55 | 3822.10±1295.65 | 473.60±269.23 |
| High dose of Embodiment 1 | 11 | 60 | 857.28±293.29 | 3566.67±501.30 | 373.03±141.25 |

5. Conclusion:
The expressions of hippocampal cAMP and PKA, serum BDNF, and hypothalamic monoamine neurotransmitters (5-HT, NE and DA) in the rats that were administered with the pharmaceutical composition of Embodiment 1 are significantly higher than those in the control group without administration (*P <0.05).

**Experiment 4: An animal experiment for the anti-depression pharmacodynamics of Embodiment 1.**
1. Animal behavior experiment:
The inventor conducted animal behavior experiments, including the tail-hang experiment on mice, the forced-swim experiment on rats, the forced-swim experiment on mice, the unpredictable long-term stimulus experiment on rats, the olfactory bulb lesion experiment on rats and others, according to the pathogenetic mechanism and clinical symptoms of depression.
1.1 The pharmaceutical composition of Embodiment 1 was intragastrically administered to mice with the dose of 20 mg/kg/d, 40 mg/kg/d and 80 mg/kg/d (the dose of 15 mg/kg/d, 30 mg/kg/d and 60 mg/kg/d for rats) for one week to determine an effect against the experimental depression, wherein the medium dose group (40 mg/kg/d for mice and 30 mg/kg/d for rats) and the positive control group (Paroxatine) demonstrated significantly shorter non-movement time for the tail-hanged mice, the forced-swim mice and the forced-swim rats. The data show a statistically significant difference compared to the control group (P < 0.05).
1.2 The pharmaceutical composition of Embodiment 1 was intragastrically administered to rats under the chronic unpredictable mild stress (CUMS) model with the dose of 15 mg/kg/d, 30 mg/kg/d and 60 mg/kg/d for 21 consecutive days. The results show that, compared to the normal group, the body weight of the CMUS model rat group gained slowly, the sucrose consumption significantly decreased (P < 0.01), the horizontal movement and vertical movement significantly decreased (P < 0.01), and the number of errors in rat jumping trials significantly increased (P < 0.01). Compared to the control group, the body weight gains of the low dose of Embodiment 1 and the positive group (Paroxetine) significantly increased, the sucrose consumption in both groups significantly increased (P < 0.01), the horizontal movement and vertical movement in both groups significantly increased (P < 0.01), and the number of errors in rat jumping trial in both groups significantly decreased (P < 0.01 for the small dose group, and P < 0.01 for the positive group (Paroxetine)).
1.3 The pharmaceutical composition of Embodiment 1 was intragastrically administered to olfactory bulb lesion model rats with the dose of 15 mg/kg/d, 30 mg/kg/d and 60 mg/kg/d for 24 consecutive days. In the open field box test, compared to the control group, the high dose of Embodiment 1 significantly improved the decrease of the horizontal movement and vertical movement caused by the olfactory bulb lesion. The positive control (Paroxetine of 3 mg/kg/d) also significantly improved the decrease of the horizontal movement and vertical movement caused by the olfactory bulb lesion. In the passive avoidance experiment, compared to the control group, the high dose and the medium dose of Embodiment 1 significantly improved the decrement of rat study and memory capacity caused by the olfactory bulb lesion.
2. The animal experiment on interaction model:
The inventor conducted animal behavior experiments, including the reserpine-induced model experiments (body temperature drop, akinesia and blepharoptosis) on mice, the serotonin-induced head shaking experiment on mice and others, according to the pathogenetic mechanism and clinical symptoms of depression.
2.1 The pharmaceutical composition of Embodiment 1 was intragastrically administered to mice with the dose of 20 mg/kg/d, 40 mg/kg/d and 80 mg/kg/d (the dose of 15 mg/kg/d, 30 mg/kg/d and 60 mg/kg/d for rats) for one week. The reserpine-induced body temperature drop, akinesia and blepharoptosis in mice can be significantly inhibited by the pharmaceutical composition of Embodiment 1, indicating that the effect against the experimental depression of Embodiment 1 may be relevant to the effect of the monoamine neurotransmitters. The amount of head-shaking of the serotonin-injected mice can be significantly increased, indicating that the anti-depression effect of Embodiment 1 may be relevant to the inhibition of monoamine oxidase (MAO). Moreover, because the experimental results show that the pharmaceutical composition of Embodiment 1 has no significant effect on the self-regulated activity of mice, the pharmaceutical composition of Embodiment 1 had no stimulation effect on the central nervous system.
3. The experimental results for the major anti-depression pharmacodynamics are summarized as follows (referring to Table 5):

| Item | Model/Method | Dose (mg/kg) | Administrated condition (route/time/frequency/ effective dose) | Major result |
|---|---|---|---|---|
| 1.1 | Forced-swim experiment on rats | 60 | Oral/7 days/2 times a day/15 mg/kg (* P < 0.05) | Significantly shortened the accumulative non-movement time in the forced-swim experiment on rats |
| | | 30 | | |
| | | 15 | | |
| 1.2 | Forced-swim experiment on mice | 80 | Oral/7 days/2 times a day /40 mg/kg (** P<0.01) | Significantly shortened the accumulative non-movement time in the forced-swim experiment on mice |
| | | 40 | | |
| | | 20 | | |
| 1.3 | Tail-hang experiment on mice | 80 | Oral/7 days/2 times a day /20 mg/kg (* P < 0.05) | Significantly shortened the accumulative non-movement time of tail-hanged mice |
| | | 40 | | |
| | | 20 | | |
| 1.4 | Self-regulated activity experiment on mice | 80 | Oral/7 days/2 times a day | No significant effect on the self-regulated activity of mice |
| | | 40 | | |
| | | 20 | | |
| 2.1 | Unpredictable long-term stimulus experiment on rats: body weight measurement | 60 | Oral/7 days/1 time a day /15 mg/kg (** P<0.01) | Significantly increased the body weight gain in the unpredictably long-term stressed rats |
| | | 30 | | |
| | | 15 | | |
| 2.2 | Unpredictable long-term stimulus experiment on rats: sucrose intake measurement | 60 | Oral/7 days/1 time a day /15 mg/kg (**P<0.01) | Significantly improved the decrease of sucrose uptake in the unpredictably long-term stressed rats |
| | | 30 | | |
| | | 15 | | |
| 2.3 | Unpredictable long-term stimulus experiment on rats: open field box test to measure self-regulated activity of the model rats | 60 | Oral/7 days/1 time a day /15 mg/kg (*P < 0.05) | Significantly improved the decrease of horizontal and vertical movements in the unpredictably long-term stressed rats |
| | | 30 | | |
| | | 15 | | |
| 2.4 | Unpredictable long-term stimulus experiment on rats: jumping test to measure the rats' behavior changes | 60 | Oral/7 days/1 time a /15 mg/kg (*P<0.05) | day Significantly decreased the number of jumping errors of the unpredictably long-term stressed rats |
| | | 30 | | |
| | | 15 | | |
| 2.5 | The determination of the monoamine neurotransmitters (NE and 5-HT, etc.) in the brain of the unpredictable long-term stimulus model rat | 60 | Oral/7 days/1 time a day /15 mg/kg **P<0.0 1) | Significantly increased the levels of NE, 5-HT and DA in the model rat's cerebral cortex |
| | | 30 | | |
| | | 15 | | |
| 3.1 | Olfactory bulb lesion experiment: open field box test to measure the self-regulated activity of mice | 60 | Oral/7 days/1 time a /30 mg/kg (*P<0.05) | day Significantly improved the rat's horizontal and vertical movements caused by the olfactory bulb lesion |
| | | 30 | | |
| | | 15 | | |
| 3.2 | Olfactory bulb lesion experiment: determination of passive avoidance experiment on rats | 60 | Oral/7 days/1 time a /30 mg/kg (*P< 0.05) | day Significantly improved the decrement of rat study and memory capability caused by the olfactory bulb lesion |
| | | 30 | | |
| | | 15 | | |
| 4.1 | Reserpine model experiment: body temperature drop on mice | 80 | Oral/7 days/2 times a day /40 mg/kg (** P<0.01) | Significantly inhibited reserpine-induced body temperature drop |
| | | 40 | | |
| | | 20 | | |
| 4.2 | Reserpine model experiment: akinesia (stupor) on mice | 80 | Oral/7 days/2 times a day /40mg/kg (** P<0.01) | Significantly inhibited reserpine-induced akinesia |
| | | 40 | | |
| | | 20 | | |
| 4.3 | Reserpine model experiment: blepharoptosis on mice | 80 | Oral/7 days/2 times a day /40 mg/kg (** P<0.01) | Significantly inhibited reserpine-induced blepharoptosis |
| | | 40 | | |
| | | 20 | | |
| 4.4 | Serotonin-induced head-shake experiment on mice | 80 | Oral/7 days/1 time a day /40 mg/kg (** P<0.01) | Significantly increased the amount of head-shaking of the serotonin-injected mice |
| | | 40 | | |
| | | 20 | | |
| Note: | 1. Embodiment 1 v.s. control *P <0.05, **P <0.01. | | | |
| | 2. Positive control: Paroxetine, 3 mg/kg/d for mice, 2 mg/kg/d for rats (In addition to that the tail-hang experiment refers to the label "**", others refer to the label "*"). | | | |

4. Conclusion: The pharmaceutical composition of Embodiment 1 has significant experimental effects against depression.

The results in the various experiments show:
(1) 8 hours after intragastrically administering the pharmaceutical composition of Embodiment 1 to rats, compared to the saline group and the Paroxetine group, the hippocampal cAMP significantly increased, the PKA activity was significantly enhanced (*P <0.05), the p-CREB level also increased, and the PDE4 activity was significantly inhibited (*P <0.05).
(2) After intragastrically administering the pharmaceutical composition of Embodiment 1 to chronically stressed mice for 10 days, the decrease of the hippocampal cAMP and PKA caused by repetitive stress significantly increased to promote the expression of the phosphorylated CREB and BDNF in the mice hippocampus.
(3) After intragastrically administering the pharmaceutical composition of Embodiment 1 to chronically stressed rats for 21 days, the decrease of the hippocampal cAMP and PKA caused by repetitive stress significantly increased to promote the expression of monoamine neurotransmitters, including serum BDNF, hypothalamic NE and 5-HT and so on , while GSC was down-regulated.
(4) The pharmaceutical composition of Embodiment 1 has significant experimental effects against depression.

The application scopes of the pharmaceutical composition of the present invention for rapidly increasing the content and availability of cAMP in vivo are described as follows.
1. The pharmaceutical composition provided for use according to the present invention can include pharmacologically acceptable additives.
2. The pharmaceutical composition used according to the present invention can be manufactured in known dosage forms, such as a powder, capsule, tablet and so on.
3. The pharmaceutical composition used according to the present invention can be manufactured as a drug, a health food or a nutrient to prevent and cure diseases caused by low availability and level of cAMP in an organism and cells, enhance the level of BDNF, enhance the cAMP/PKA/CREB signaling pathway in cells, enhance the levels of neurotransmitters including DA, NE and 5-HT, and enhance memory.

There are further embodiments disclosed as follows.

Embodiment 1. In a pharmaceutical composition provided for use in a method of treating neurodegenerative diseases and diseases caused by low cAMP expression in a patient, wherein said neurodegenerative diseases are selected from a group comprising amnesia, dementia, Alzheimer's disease and Parkinson's disease, and said diseases caused by low cAMP expression are selected from a group comprising heart failure, psoriasis and cancers, said pharmaceutical composition is characterized in that it comprises: a first main component including ginsenosides Rg1, Rb1 and Re; a second main component including a glycyrrhiza related acid selected from a group consisting of a glycyrrhizic acid, a glycyrrhetinic acid and a combination thereof; and a third main component including a jujuba cyclic adenosine monophosphate.

Embodiment 2. In the pharmaceutical composition used according to Embodiment 1, the pharmaceutical composition includes 2 - 26 parts by weight of the ginsenosides Rg1 and Rb1, 3 - 48 parts by weight of the glycyrrhiza related acid, and 0.002 - 0.5 parts by weight of the jujuba cyclic adenosine monophosphate.

Embodiment 3. In the pharmaceutical composition used according to Embodiment 1 or 2, the pharmaceutical composition includes 4 - 13 parts by weight of the ginsenosides Rg1 and Rb1, 5 - 16 parts by weight of the glycyrrhiza related acid, and 0.01 - 0.1 parts by weight of the jujuba cyclic adenosine monophosphate.

Embodiment 4. In the phannaceutical composition used according to one of Embodiments 1-3, the pharmaceutical composition includes a pharmacologically acceptable carrier, an additive or a combination thereof.

Embodiment 5. In the pharmaceutical composition used according to one of Embodiments 1-4, the pharmaceutical composition has a dosage form being one selected from a group consisting of a tablet, a capsule, a powder, a pill, a dust, a solution, a microcapsule, a suspension, an emulsion, a particle, a dropping pill, a roll and a pharmacological oral medication dosage.

Embodiment 6. In the pharmaceutical composition used according to one of Embodiments 1-5, the pharmaceutical composition is manufactured as a drug, a health food or a nutrient for use in a method of treating neurodegenerative diseases and diseases caused by low cAMP expression in a patient as defined in Embodiment 1.

Embodiment 7. In the pharmaceutical composition used according to one of Embodiments 1-6, the pharmaceutical composition further includes a fourth main component selected from a ginger, a ginger extract or a combination thereof.

Embodiment 8. In a pharmaceutical composition used according to one of Embodiments 1-7, including: a first main component including ginsenosides Rg1, Rb1 and Re; a second main component including a glycyrrhiza related acid selected from a group consisting of a glycyrrhizic acid, a glycyrrhetinic acid and a combination thereof; and a third main component including a jujuba cyclic adenosine monophosphate.

Embodiment 9. In a method for preparing a pharmaceutical composition which is for the use in a method of treating neurodegenerative diseases and diseases caused by low cAMP expression in a patient, wherein said neurodegenerative diseases are selected from a group comprising amnesia, dementia, Alzheimer's disease and Parkinson's disease, and said diseases caused by low cAMP expression are selected from a group comprising heart failure, psoriasis and cancers, and wherein said method comprises the steps of: providing a first main component, wherein the first main component includes ginsenosides Rg1, Rb1 and Re; providing a second main component, wherein the second main component includes a glycyrrhiza related acid being one selected from a group consisting of a glycyrrhizic acid, a glycyrrhetinic acid and a combination thereof; and providing a third main component, wherein the third main component includes a jujuba cyclic adenosine monophosphate, to manufacture said pharmaceutical composition.

### Industrial Applicability

1. The pharmaceutical composition provided for the use according to the present invention can include pharmacologically acceptable additives.
2. The pharmaceutical composition provided for the use according to the present invention can be manufactured in known dosage forms, such as a powder, capsule or tablet.
3. The pharmaceutical composition provided for the use according to the present invention can be manufactured as a drug, a health food or a nutrient for use in a method of treating neurodegenerative diseases and diseases caused by low cAMP expression in a patient as defined herein.

## Claims

1. A pharmaceutical composition for use in a method of treating neurodegenerative diseases and diseases caused by low cAMP expression in a patient, wherein said neurodegenerative diseases are selected from a group comprising amnesia, dementia, Alzheimer's disease and Parkinson's disease, and said diseases caused by low cAMP expression are selected from a group comprising psoriasis and cancers, and wherein said pharmaceutical composition is **characterized in that** it comprises:
- a first main component comprising ginsenosides Rg1, Rb1 and Re;
- a second main component comprising a glycyrrhiza related acid being one selected from a group consisting of a glycyrrhizic acid, a glycyrrhetinic acid and a combination thereof; and
- a third main component comprising a jujuba cyclic adenosine monophosphate (jujuba cAMP).

2. The pharmaceutical composition for use according to Claim 1, **characterized in that** said pharmaceutical composition comprises 2 - 26 parts by weight of the ginsenosides Rg1, Rb1 and Re, 3 - 48 parts by weight of the glycyrrhiza related acid, and 0.002 - 0.5 parts by weight of the jujuba cyclic adenosine monophosphate.

3. The pharmaceutical composition for use according to Claim 1 or 2, **characterized in that** the pharmaceutical composition comprises 4 - 13 parts by weight of the ginsenosides Rg1, Rb1 and Re, 5 - 16 parts by weight of the glycyrrhiza related acid, and 0.01 - 0.1 parts by weight of the jujuba cyclic adenosine monophosphate.

4. The pharmaceutical composition for use according to any of the Claims 1-3, **characterized in that** the ginsenosides Rg1, Rb1 and Re are extracted from a ginseng, the glycyrrhiza related acid is extracted from a liquorice, and the jujuba cAMP is extracted from a jujuba.

5. The pharmaceutical composition for use according to any of the Claims 1-4, **characterized in that** the pharmaceutical composition comprises a pharmacologically acceptable carrier, an additive or a combination thereof.

6. The pharmaceutical composition for use according to any of the Claims 1-5, **characterized in that** the pharmaceutical composition has a dosage form selected from a group consisting of a tablet, a capsule, a powder, a pill, a dust, a solution, a microcapsule, a suspension, an emulsion, a particle, a dropping pill, a roll and a pharmacological oral medication dosage.

7. The pharmaceutical composition for use according to any of the Claims 1-6, **characterized in that** the pharmaceutical composition is manufactured as a drug, a health food or a nutrient for use in a method of treating neurodegenerative diseases and diseases caused by low cAMP expression in a patient as defined in claim 1..

8. The pharmaceutical composition for use according to any of the Claims 1-7, **characterized in that** the pharmaceutical composition further comprises a fourth main component selected from a group consisting of a ginger, a ginger extract and a combination thereof.

9. The pharmaceutical composition for use according to Claim 8, **characterized in that** the ginger extract is a ginger water extract.

10. A method for preparing a pharmaceutical composition, which is for the use in a method of treating neurodegenerative diseases and diseases caused by low cAMP expression in a patient, wherein said neurodegenerative diseases are selected from a group comprising amnesia, dementia, Alzheimer's disease and Parkinson's disease, and said diseases caused by low cAMP expression are selected from a group comprising psoriasis and cancers, and wherein said method comprises the steps of:
- providing a first main component, wherein the first main component comprises ginsenosides Rg1, Rb1 and Re;
- providing a second main component, wherein the second main component comprises a glycyrrhiza related acid being one selected from a group consisting of a glycyrrhizic acid, a glycyrrhetinic acid and a combination thereof; and
- providing a third main component, wherein the third main component comprises a jujuba cyclic adenosine monophosphate (jujuba cAMP), to manufacture said pharmaceutical composition.

11. The method according to Claim 10, **characterized in that** the ginsenosides Rg1, Rb1 and Re are extracted from a ginseng, the glycyrrhiza related acid is extracted from a liquorice, and the jujuba cAMP is extracted from a jujuba.

12. The method according to Claim 10 or 11, further **characterized by** comprising a step of providing a fourth main component, wherein said fourth main component is selected from a group consisting of a ginger, a ginger extract and a combination thereof.

13. The method according to Claim 12, **characterized in that** the ginger is extracted by water to obtain the ginger extract.

## Patentansprüche

1. Pharmazeutische Zusammensetzung zur Verwendung in einem Verfahren zur Behandlung von neurodegenerativen Erkrankungen und Erkrankungen, die durch eine geringe Expression von cAMP ausgelöst werden bei einem Patienten, wobei die neurodegenerativen Erkrankungen ausgewählt sind aus einer Gruppe umfassend Amnesie, Demenz, Morbus Alzheimer und Morbus Parkinson, und wobei die Erkrankungen, die durch eine geringe Expression von cAMP ausgelöst werden, ausgewählt sind aus einer Gruppe bestehend aus Psoriasis und Krebserkrankungen, und wobei die pharmazeutische Zusammensetzung **dadurch gekennzeichnet ist, dass** sie Folgendes umfasst:
- eine erste Hauptkomponente, umfassend die Ginsenoside Rg1, Rb1 und Re;
- eine zweite Hauptkomponente, umfassend eine Glycyrrhiza-ähnliche Säure, ausgewählt aus einer Gruppe bestehend aus einer Glycyrrhizinsäure, einer Glycyrrhetinsäure und einer Kombination davon; und
- eine dritte Hauptkomponente, umfassend ein Jujube zyklisches Adenosinmonophosphat (Jujube-cAMP).

2. Pharmazeutische Zusammensetzung zur Verwendung nach Anspruch 1, **dadurch gekennzeichnet, dass** die pharmazeutische Zusammensetzung 2 bis 26 Gewichtsteile der Ginsenoside Rg1, Rb1 und Re, 3 bis 48 Gewichtsteile der Glycyrrhiza-ähnlichen Säure, und 0,002 bis 0,5 Gewichtsteile des Jujube zyklischen Adenosinmonophosphats umfasst.

3. Pharmazeutische Zusammensetzung zur Verwendung nach Anspruch 1 oder 2, **dadurch gekennzeichnet, dass** die pharmazeutische Zusammensetzung 4 bis 13 Gewichtsteile der Ginsenoside Rg1, Rb1 und Re, 5 bis 16 Gewichtsteile der Glycyrrhiza-ähnlichen Säure, und 0,01 bis 0,1 Gewichtsteile des Jujube zyklischen Adenosinmonophosphats umfasst.

4. Pharmazeutische Zusammensetzung zur Verwendung nach einem der Ansprüche 1 bis 3, **dadurch gekennzeichnet, dass** die Ginsenoside Rg1, Rb1 und Re aus einem Ginseng extrahiert werden, die Glycyrrhiza-ähnliche Säure aus einem Süßholz extrahiert wird, und das Jujube-cAMP aus einer Jujube extrahiert wird.

5. Pharmazeutische Zusammensetzung zur Verwendung nach einem der Ansprüche 1 bis 4, **dadurch gekennzeichnet, dass** die pharmazeutische Zusammensetzung einen pharmakologisch akzeptablen Trägerstoff, ein Additiv oder eine Kombination davon umfasst.

6. Pharmazeutische Zusammensetzung zur Verwendung nach einem der Ansprüche 1 bis 5, **dadurch gekennzeichnet, dass** die pharmazeutische Zusammensetzung eine Darreichungsform hat, ausgewählt aus einer Gruppe bestehend aus einer Tablette, einer Kapsel, einem Pulver, einer Pille, einem Staub, einer Lösung, einer Mikrokapsel, einer Suspension, einer Emulsion, einem Partikel, einer Tropfpille, einer Rolle und einer pharmakologischen oralen Arzneimitteldosis.

7. Pharmazeutische Zusammensetzung zur Verwendung nach einem der Ansprüche 1 bis 6, **dadurch gekennzeichnet, dass** die pharmazeutische Zusammensetzung als ein Arzneimittel, eine Gesundheitskost oder ein Nährstoff hergestellt wird zur Verwendung in einem Verfahren zur Behandlung von neurodegenerativen Erkrankungen und Erkrankungen, die durch eine geringe Expression von cAMP ausgelöst werden bei einem Patienten wie in Anspruch 1 definiert.

8. Pharmazeutische Zusammensetzung zur Verwendung nach einem der Ansprüche 1 bis 7, **dadurch gekennzeichnet, dass** die pharmazeutische Zusammensetzung weiterhin eine vierte Hauptkomponente umfasst, ausgewählt aus einer Gruppe bestehend aus Ingwer, einem Ingwerextrakt und einer Kombination davon.

9. Pharmazeutische Zusammensetzung zur Verwendung nach Anspruch 8, **dadurch gekennzeichnet, dass** das Ingwerextrakt ein Ingwerwasserextrakt ist.

10. Verfahren zur Herstellung einer pharmazeutischen Zusammensetzung zur Verwendung in einem Verfahren zur Behandlung von neurodegenerativen Erkrankungen und Erkrankungen, die durch eine geringe Expression von cAMP ausgelöst werden bei einem Patienten, wobei die neurodegenerativen Erkrankungen ausgewählt sind aus einer Gruppe umfassend Amnesie, Demenz, Morbus Alzheimer und Morbus Parkinson, und wobei die Erkrankungen, die durch eine geringe Expression von cAMP ausgelöst werden, ausgewählt sind aus einer Gruppe bestehend aus Psoriasis und Krebserkrankungen, und wobei das Verfahren die folgenden Schritte umfasst:
- Bereitstellen einer ersten Hauptkomponente, wobei die erste Hauptkomponente die Ginsenoside Rg1, Rb1 und Re umfasst;
- Bereitstellen einer zweiten Hauptkomponente, wobei die zweite Hauptkomponente eine Glycyrrhiza-ähnliche Säure umfasst, ausgewählt aus einer Gruppe bestehend aus einer Glycyrrhizinsäure, einer Glycyrrhetinsäure und einer Kombination davon; und
- Bereitstellen einer dritten Hauptkomponente, wobei die dritte Hauptkomponente ein Jujube zyklisches Adenosinmonophosphat (Jujube-cAMP) umfasst, um die pharmazeutische Zusammensetzung herzustellen.

11. Verfahren nach Anspruch 10, **dadurch gekennzeichnet, dass** die Ginsenoside Rg1, Rb1 und Re aus einem Ginseng extrahiert werden, die Glycyrrhiza-ähnliche Säure aus einem Süßholz extrahiert wird, und das Jujube-cAMP aus einer Jujube extrahiert wird.

12. Verfahren nach Anspruch 10 oder 11, weiterhin **dadurch gekennzeichnet, dass** es einen Schritt des Bereitstellens einer vierten Hauptkomponente umfasst, wobei die vierte Hauptkomponente ausgewählt ist aus einer Gruppe bestehend aus Ingwer, einem Ingwerextrakt und einer Kombination davon.

13. Verfahren nach Anspruch 12, **dadurch gekennzeichnet, dass** der Ingwer durch Wasser extrahiert wird, um das Ingwerextrakt zu erhalten.

## Revendications

1. Composition pharmaceutique pour l'utilisation dans un procédé de traitement de maladies neurodégénératives et de maladies provoquées par une faible expression d'AMPc chez un patient, dans laquelle lesdites maladies neurodégénératives sont choisies dans un groupe comprenant l'amnésie, la démence, la maladie d'Alzheimer et la maladie de Parkinson, et lesdites maladies provoquées par une faible expression d'AMPc sont choisies dans un groupe comprenant le psoriasis et les cancers, et où ladite composition pharmaceutique est **caractérisée en ce qu'**elle comprend :
- un premier composant principal comprenant les ginsénosides Rg1, Rb1 et Re ;
- un deuxième composant principal comprenant un acide associé à glycyrrhiza qui est un choisi dans un groupe constitué par un acide glycyrrhizique, un acide glycyrrhétinique et une de leurs combinaisons ; et
- un troisième composant principal comprenant une adénosine monophosphate cyclique de jujuba (AMPc de jujuba).

2. Composition pharmaceutique pour l'utilisation selon la revendication 1, **caractérisée en ce que** ladite composition pharmaceutique comprend 2 à 26 parties en poids des ginsénosides Rg1, Rb1 et Re, 3 à 48 parties en poids de l'acide associé à glycyrrhiza et 0,002 à 0,5 parties en poids de l'adénosine monophosphate cyclique de jujuba.

3. Composition pharmaceutique pour l'utilisation selon la revendication 1 ou 2, **caractérisée en ce que** la composition pharmaceutique comprend 4 à 13 parties en poids des ginsénosides Rg1, Rb1 et Re, 5 à 16 parties en poids de l'acide associé à glycyrrhiza et 0,01 à 0,1 parties en poids de l'adénosine monophosphate cyclique de jujuba.

4. Composition pharmaceutique pour l'utilisation selon l'une quelconque des revendications 1 à 3, **caractérisée en ce que** les ginsénosides Rg1, Rb1 et Re sont extraits d'un ginseng, l'acide associé à glycyrrhiza est extrait d'un réglisse et l'AMPc de jujuba est extrait d'un jujuba.

5. Composition pharmaceutique pour l'utilisation selon l'une quelconque des revendications 1 à 4, **caractérisée en ce que** la composition pharmaceutique comprend un véhicule pharmacologiquement acceptable, un additif ou une de leurs combinaisons.

6. Composition pharmaceutique pour l'utilisation selon l'une quelconque des revendications 1 à 5, **caractérisée en ce que** la composition pharmaceutique a une forme posologique choisie dans un groupe constitué par un comprimé, une capsule, une poudre, une pilule, une poussière, une solution, une microcapsule, une suspension, une émulsion, une particule, une pilule de largage, un rouleau et une posologie de médication orale pharmacologique.

7. Composition pharmaceutique pour l'utilisation selon l'une quelconque des revendications 1 à 6, **caractérisée en ce que** la composition pharmaceutique est fabriquée comme un médicament, un aliment de santé ou un élément nutritif pour l'utilisation dans un procédé de traitement de maladies neurodégénératives et de maladies provoquées par une faible d'expression d'AMPc chez un patient tel que défini dans la revendication 1.

8. Composition pharmaceutique pour l'utilisation selon l'une quelconque des revendications 1 à 7, **caractérisée en ce que** la composition pharmaceutique comprend en outre un quatrième composant principal choisi dans un groupe constitué par un gingembre, un extrait de gingembre et une de leurs combinaisons.

9. Composition pharmaceutique pour l'utilisation selon la revendication 8, **caractérisée en ce que** l'extrait de gingembre est un extrait aqueux de gingembre.

10. Procédé de préparation d'une composition pharmaceutique, qui est pour l'utilisation dans un procédé de traitement de maladies neurodégénératives et de maladies provoquées par une faible expression d'AMPc chez un patient, où lesdites maladies neurodégénératives sont choisies dans un groupe comprenant l'amnésie, la démence, la maladie d'Alzheimer et la maladie de Parkinson, et lesdites maladies provoquées par une faible expression d'AMPc sont choisies dans un groupe comprenant le psoriasis et les cancers, et où ledit procédé comprend les étapes de :
- fourniture d'un premier composant principal, où le premier composant principal comprend les ginsénosides Rg1, Rb1 et Re ;
- fourniture d'un deuxième composant principal, où le deuxième composant principal comprend un acide associé à glycyrrhiza choisi dans un groupe constitué par un acide glycyrrhizique, un acide glycyrrhétinique et une de leurs combinaisons ; et
- fourniture d'un troisième composant principal, où le troisième composant principal comprend une adénosine monophosphate cyclique de jujuba (AMPc de jujuba), pour fabriquer ladite composition pharmaceutique.

11. Procédé selon la revendication 10, **caractérisé en ce que** les ginsénosides Rg1n Rb1 et Re sont extraits d'un ginseng, l'acide associé à glycyrrhiza est extrait d'un réglisse et l'AMPc de jujuba est extrait d'un jujuba.

12. Procédé selon la revendication 10 ou 11, caractérisé en outre comme comprenant une étape de fourniture d'un quatrième composant principal, où ledit quatrième composant principal est choisi dans un groupe constitué par un gingembre, un extrait de gingembre et une de leurs combinaisons.

13. Procédé selon la revendication 12, **caractérisé en ce que** le gingembre est extrait par de l'eau pour obtenir l'extrait de gingembre.
